# EUROPÄISCHE PATENTSCHRIFT

(11) **EP 0 523 461 B1**
(45) Veröffentlichungstag und Bekanntmachung des Hinweises auf die Patenterteilung: **22.01.1997**
(21) Anmeldenummer: 92111238.9
(22) Anmeldetag: 02.07.1992
(51) Int. Cl.: C07C 67/03, C07C 69/614, C07K 1/00

(54) **Verfahren zur Verseifung von Aminosäure-/Peptidestern**
Process for the saponification of aminoacid-/peptide esters
Procédé de saponification d'esters d'aminoacide-/peptides

(30) Priorität: 15.07.1991 DE 4123408; 22.07.1991 DE 4124283
(43) Veröffentlichungstag der Anmeldung: 20.01.1993
(60) Teilanmeldung: 95108067.0
(73) Patentinhaber: DEGUSSA AG, 60311 Frankfurt (DE)
(72) Erfinder: Drauz, Karl-Heinz, Dr., W-6463 Freigericht 1 (DE); Müller, Thomas, Dr., W-6000 Frankfurt 90 (DE); Kottenhahn, Matthias, Dr., W-6450 Hanau 7 (DE); Seebach, Dieter, Prof. Dr., CH-8044 Zürich (CH); Thaler, Adrian, Dr., CH-6312 Steinhausen (CH)

(56) Entgegenhaltungen:
- EP-A- 0 110 629
- EP-A- 0 150 962

## Beschreibung

Die Erfindung betrifft ein Verfahren zur Verseifung von Carbonsäurederivaten. Die Erfindung betrifft insbesondere ein Verfahren zur Verseifung eines Peptidesters in wäßrigem Medium in Gegenwart einer Metallverbindung als Base. In der Literatur wurden bereits verschiedene Verfahren zur Verseifung der genannten Verbindungen beschrieben. Hierbei wird z. B. unter stark sauren oder basischen Bedingungen mit Enzymen (D. Seebach, Angew. Chem. 1990, 102, 1363) oder mit Ionenaustauscherharzen (W. Pereira, V. Close, W. Patton, B. Halpfern, J. Org. Chem. 1969, 34 2032) gearbeitet. Weiterhin wurde ein Verfahren zur Umesterung bzw. Verseifung von Esterderivaten unter Verwendung der Amidinbase DBU in Lösung (EP-en 0 110 629, 0 150 962) oder polymergebunden (T. Ishikawa, Y. Ohsumi, T. Kawai, Bull. Chem. Soc. Jpn. 1990, 63, 819) beschrieben.

Aus J. Org. Chem. 38 (1973), 1223 - 1225 ist die Spaltung von Carbonsäuremethylestern mit DBU bei 165 °C über 48 h bekannt. Solche Bedingungen sind für die meisten Esterspaltungen zu drastisch, dies gilt insbesondere für optisch aktive Ester, die in α-Stellung zur Estergruppe ein Chiralitätszentrum besitzen.

Aus CA 114, 186011 (1991) und Tetrahedron Letters 21 (1980), 1181 - 1184 ist eine β-Eliminierung mit DBU oder DBN bekannt. Wie bei β-Eliminierungen allgemein üblich und hier auch erwähnt, kann prinzipiell fast jede Base, z. B. auch Kaliumhydroxid, eingesetzt werden. Allgemein verwendbare milde Reaktionsbedingungen sind hier nicht beschrieben.

Aus "Aminosäuren, Peptide, Proteine", Verlag Chemie Weinheim 1982, Seite 47ff ist weiterhin ein Verfahren zur alkalischen Hydrolyse von Peptiden und Proteinen mit Ba(OH)₂ unter hohem Druck bekannt. Ein Nachteil dieses Verfahrens ist die partielle bis vollständige Racemisierung der Aminosäuren.

Alle bisher genannten Verfahren, mit Ausnahme der enzymatischen Methode, benötigen im Normalfall erhöhte Temperaturen, extreme pH-Werte und/oder lange Reaktionszeiten. Für empfindliche Esterderivate, die z. B. weitere funktionelle Gruppen tragen oder ein oder mehrere chirale C-Atome besitzen, insbesondere für solche Verbindungen, die in α-Position zur Esterfunktion ein chirales C-Atom besitzen, sind die meisten der genannten Methoden aufgrund der Reaktionsbedingungen ungeeignet.

Aufgabe der vorliegenden Erfindung ist daher ein System zur Umsetzung, insbesondere zur Verseifung von Säurederivaten, das so milde Reaktionsbedingungen hat, daß insbesondere optisch aktive und Biomoleküle wie Peptide oder Aminosäuren eingesetzt werden können.

Diese Aufgabe wird gelöst durch den kombinierten Einsatz von einer Amidinbase und einer Metallverbindung, insbesondere bei der Verseifung. Bei der Verseifung von Peptidestern zeigte es sich, daß hier auch ohne die Amidinbase einzig schon mit Lithiumhydroxid unter milden Bedingungen gespalten werden kann.

Die Anmeldung beansprucht somit ein Verfahren zur Verseifung eines Aminosäureesters oder Peptidesters in wässrigem Medium mittels einer Base, wobei die Verseifung mit mindestens 0,1 molarer Menge Lithiumhydroxid als Base ausgeführt wird.

Bevorzugt wird die Verseifung in Gegenwart einer Metallverbindung, ausgewählt aus Salzen von Lithium, Magnesium oder Caesium, ausgeführt.

So können z. B. empfindliche Peptidester ohne Racemisierung am α-C-Atom der C-terminal positionierten Aminosäure verseift werden. Dabei bleiben eventuell geschützte Seitenkettenfunktionalitäten, die keine Esterfunktion enthalten, unberührt. Die Verseifung war dagegen mit wässriger NaOH nicht zersetzungsfrei und führte zur Racemisierung.

Zur Verseifung der Säurederivate, bevorzugt ein Ester, wird nach dem erfindungsgemäßen Verfahren üblicherweise wie folgt gearbeitet:

Das Säurederivat wird in einem Lösungsmittel, bevorzugt werden Ether wie THF oder Dioxan eingesetzt, gelöst oder suspendiert. Nach Zusatz von Wasser (1 +, bevorzugt 10 - 100fach molarer Menge), der Amidinbase, z. B. DBU oder DBN, die in 1 - 10 molarer Menge, bevorzugt in 1 - 4 molarer Menge, eingesetzt wird, und Zusatz der Metallverbindung, bevorzugt werden Salze des Lithiums, Magnesiums oder Caesiums in 0,1 - 20 molarer Menge, insbesondere in 2 - 10 molarer Menge, wird bei Temperaturen zwischen -20 ° und 65 °C umgesetzt. Die Reihenfolge der Zugabe ist beliebig. Bei racemisierungsempfindlichen Derivaten wird bevorzugt zwischen -20 ° und 30 °C bei kurzen Reaktionszeiten umgesetzt.

Als Metallverbindungen werden bei den obigen Verfahren Halogenide, insbesondere Bromid oder auch Chlorid, Hydroxid, Perchlorat, Acetat, Sulfat oder Carbonat bevorzugt.

Die Metallverbindung, besonders geeignet sind Lithium- und ferner Magnesium- oder Cäsiumsalze, wird üblicherweise in 0,1 - 20 molarer Menge eingesetzt. Besonders günstig sind 2 - 10 molare Mengen der Metallverbindung, jeweils bezogen auf das Säurederivat.

Bei den Untersuchungen, die zur vorliegenden Erfindung führten, zeigte es sich auch, daß in einigen Fällen allein Lithiumhydroxid, oder ein anderes Lithiumsalz und eine Base, so daß Lithium- und Hydroxidionen in der Reaktionslösung vorliegen, zur Verseifung von Aminosäure- oder Peptidestern eingesetzt werden kann. Hierbei ist eine 1,0 - 20 molare Menge an Lithiumhydroxid geeignet, bevorzugt ist eine 2 - 20 molare Menge an Lithiumhydroxid gegenüber der zu verseifenden Verbindung. Liegt das Lithiumhydroxid in einem Puffersystem vor bzw. ist eine Hilfsbase zugegen, dann kann die Lithiumverbindung auch schon in 0,1 molarer Menge eingesetzt werden. Die entsprechende freie Säure wird dann durch die Hilfsbase und das Puffersystem neutralisiert, so daß der alkalische Charakter der Reaktionslösung zur Verseifung erhalten bleibt.

Anhand des nachfolgenden Beispiels wird die Erfindung näher ausgeführt.

### Beispiel 1

### Ac-D-Nal-D-p-Cl-Phe-D-Pal-Ser-Tyr-D-Cit-Leu-OH

250 mg (0,223 mmol) Ac-D-Nal-D-p-Cl-Phe-D-Pal-Ser-Tyr-D-Cit-Leu-OMe wurden in 15 ml THF suspendiert, die Suspension mit 1 ml Wasser und der Lösung von 11,2 mg (0,468 mmol) LiOH in 1 ml Wasser versetzt und die Reaktionsmischung 4 Stunden bei Raumtemperatur gerührt. Danach war laut HPLC kein Edukt mehr vorhanden. Man brachte die Reaktionslösung mit 1N Salzsäure auf einen pH von 4, entfernte das THF im Vakuum, verdünnte den Rückstand mit 15 ml Wasser und saugte ab. Das Produkt wurde mit 30 ml Acetonitril bei 80 °C heiß digeriert, erneut abgesaugt und getrocknet. Man erhielt schließlich 220 mg (90 %) Ac-D-Nal-D-p-Cl-Phe-D-Pal-Ser-Tyr-D-Cit-Leu-OH, laut HPLC mit einer Reinheit von 98,5 %. Im ¹H-NMR-Spektrum war das Signal des Methylesters bei 3,6 ppm nicht mehr vorhanden, ansonsten entsprach das Spektrum einem Vergleichs spektrum von auf unabhängigem Wege hergestellten Ac-D-Nal-D-p-Cl-Phe-D-Pal-Ser-Tyr-D-Cit-Leu-OH. Ein GC-Racemattest zeigte keine signifikante Racemisierung am Leucin (D-Leu 0,5 %).

## Patentansprüche

1. Verfahren zur Verseifung eines Aminosäureesters oder Peptidesters in wässrigem Medium mittels einer Base,
**dadurch gekennzeichnet,**
daß die Verseifung mit mindestens 0,1 molarer Menge Lithiumhydroxid als Base ausgeführt wird.

2. Verfahren nach Anspruch 1,
**dadurch gekennzeichnet,**
daß es in Gegenwart einer Metallverbindung, ausgewählt aus Salzen von Lithium, Magnesium oder Caesium, ausgeführt wird.

3. Verfahren nach Anspruch 2,
**dadurch gekennzeichnet,**
daß die Metallverbindung in 0,1 - 20 molarer Menge eingesetzt wird.

4. Verfahren nach einem der vorstehenden Ansprüche,
**dadurch gekennzeichnet,**
daß es bei einer Temperatur im Bereich -20 °C bis +65 °C durchgeführt wird.

5. Verfahren nach einem der vorstehenden Ansprüche 2 bis 4,
**dadurch gekennzeichnet,**
daß die Metallverbindung ein Bromid, Chlorid, Hydroxid, Perchlorat, Acetat, Sulfat oder Carbonat ist.

6. Verfahren nach einem der vorstehenden Ansprüche,
**dadurch gekennzeichnet,**
daß zur Kompensation der entstehenden freien Säure eine Hilfsbase mitverwendet oder ein Puffersystem eingesetzt wird.

7. Verfahren nach einem der vorstehenden Ansprüche,
**dadurch gekennzeichnet,**
daß Lithiumhydroxid in 1,0 bis 20 molarer Menge eingesetzt wird.

## Claims

1. A method of saponifying an amino acid ester or peptide ester in an aqueous medium, using a base,
characterised in that saponification is brought about by using a base in the form of an at least 0.1 molar quantity of lithium hydroxide.

2. A method according to claim 1, characterised in that it is performed in the presence of a metal compound selected from salts of lithium, magnesium or caesium.

3. A method according to claim 2, characterised in that the metal compound is used in a 0.1 - 20 molar proportion.

4. A method according to any of the preceding claims, characterised in that it is carried out at a temperature in the range from -20°C to +65°C.

5. A method according to any of the preceding claims 2 to 4, characterised in that the metal compound is a bromide, chloride, hydroxide, perchlorate, acetate, sulphate or carbonate.

6. A method according to any of the preceding claims, characterised in that an auxiliary base or a buffer system is used in order to compensate the free acids which are evolved.

7. A method according to any of the preceding claims, characterised in that lithium hydroxide in a 1.0 to 20 molar proportion is used.

## Revendications

1. Procédé de saponification d'un ester d'acide aminé ou d'un ester de peptide en milieu aqueux au moyen d'une base, caractérisé en ce que la saponification est réalisée avec une quantité d'au moins 0,1 mole d'hydroxyde de lithium à titre de base.

2. Procédé selon la revendication 1, caractérisé en ce qu'il est effectué en présence d'un composé métallique choisi parmi les sels de lithium, de magnésium ou de césium.

3. Procédé selon la revendication 2, caractérisé en ce que le composé métallique est utilisé en quantité de 0,1 à 20 moles.

4. Procédé selon l'une quelconque des revendications précédentes, caractérisé en ce qu'il est réalisé à une température dans la plage de -20°C à +65°C.

5. Procédé selon l'une quelconque des revendications 2 à 4, caractérisé en ce que le composé métallique est un bromure, un chlorure, un hydroxyde, un perchlorate, un acétate, un sulfate ou un carbonate.

6. Procédé selon l'une quelconque des revendications précédentes, caractérisé en ce qu'on utilise conjointement, pour compenser l'acide libre produit, une base auxiliaire ou on emploie un système tampon.

7. Procédé selon l'une quelconque des revendications précédentes, caractérisé en ce qu'on utilise de l'hydroxyde de lithium en quantité de 1,0 à 20 moles.
